# EUROPEAN PATENT SPECIFICATION

(11) **EP 4 547 279 B1**
(45) Date of publication and mention of the grant of the patent: **04.11.2026**
(21) Application number: 23736011.0
(22) Date of filing: 23.06.2023
(51) Int. Cl.: A61K 47/02, A61K 9/00

(54) **ORELLANINE FORMULATION**
ORELLANINFORMULIERUNG
FORMULATION D'ORELLANINE

(30) Priority: 01.07.2022 US 202263357757 P
(43) Date of publication of application: 07.05.2025
(73) Proprietor: Oncorena AB, 22381 Lund (SE)
(72) Inventor: YAMEOGO, Olivier, 63200 Riom (FR); COISSARD, Emmanuelle, 63200 Riom (FR); FALCK, Pål, 0854 Oslo (NO)
(74) Representative: Finnegan Europe LLP
(86) International application number: PCT/EP2023/067195
(87) International publication number: WO 2024/002909

(56) References cited:
- WO-A1-2010/040750

## Description

### FIELD OF INVENTION

The present invention relates to a pharmaceutical formulation comprising Orellanine.

### BACKGROUND OF THE INVENTION

Orellanine (see Formula I below) is a selective renal toxin occurring in relatively large amounts in several fungal species of the Cortinarius family.

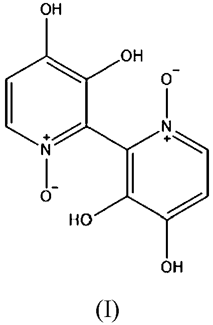

WO 2010/040750 discloses that Orellanine may be used in the treatment of renal cell carcinoma.

Orellanine was taken up in human renal cancer cells and killed them with great efficiency whether they were derived from a primary tumor or from metastatic tumor tissue. The cell death progressed for many days after transient exposure to Orellanine, indicating that the toxin was actively taken up and retained by the cells.

Orellanine drug products are being developed as treatments for renal cancer. It is therefore desirable to provide pharmaceutical formulations of Orellanine.

The present inventors have sought to provide stable formulations of Orellanine that are suitable for administration by intravenous injection.

### SUMMARY OF THE INVENTION

Accordingly, the present invention provides a pharmaceutical aqueous solution formulation comprising
from 0.1 to 40 mg/ml of Orellanine; and
a buffer;
wherein the pH of the formulation is in the range of 7 to 8, and
wherein the concentration of the buffer is from 40 to 80 mM.

The present inventors have found that such formulations are stable and suitable for administration by intravenous injection.

The scope of the invention is defined in the appended claims.

### DETAILED DESCRIPTION OF THE INVENTION

The pharmaceutical formulation is an aqueous formulation. Water for injection (WFI) may be used in aqueous formulations that are to be administered by intravenous injection.

The amount of Orellanine in the formulation is from 0.1 to 40 mg/ml and may be from 0.5 to 30 gm/ml, from 1 to 20 mg/ml of Orellanine, from 5 to 15 mg/ml of Orellanine, from 8 to 12 mg/ml of Orellanine, from 9 to 11 mg/ml of Orellanine, or about 10 mg/ml of Orellanine. In an embodiment, the formulation comprises 10 mg/ml of Orellanine. It is possible to determine the amount of Orellanine in a formulation by conventional methods including High-Performance Liquid Chromatography (HPLC).

The inventors have found that the solubility of Orellanine is pH dependent. Outside the preferred pH range the solubility of Orellanine is low and it is not possible to provide the concentration of Orellanine that is preferred for a pharmaceutical formulation for administration by intravenous injection. The pH of the formulation is in the range of 7 to 8, and may be in the range of 7.1 to 7.6, or in the range of 7.2 to 7.5, or in the range of 7.3 to 7.4. In an embodiment the pH is about 7.4 (from 7.39 to 7.41). pH may be determined by standard methods such as in accordance with Ph. Eur. 2.2.3.

The inventors have found that the concentration of the buffer has an impact on the solubility of Orellanine. The buffer concentration is from 40 to 80 mM, and may be from 40 to 60 mM.

The buffer may be a phosphate buffer such as NaH₂PO₄ - Na₂HPO₄ or Citrate - Na₂HPO₄.

The buffer ensures that the pH is within the chosen range. Further adjustment of the pH may be carried out by addition of base or acid, such as addition of sodium hydroxide or hydrochloric acid, e.g. with sodium hydroxide or hydrochloric acid at 0.2M.

The inventors have found that the osmolality of the formulation affects the solubility of the Orellanine. The osmolality of the formulation may be greater than 200 mOsm, or may be in the range of from 250 to 350 mOsm. Osmolality may be determined by standard methods, such as in accordance with Ph. Eur. 2.2.35. In one embodiment of the invention, the osmolality of the formulation is such that it is within the isotonic range, i.e. the formulation has an isotonicity that is similar to the isotonicity of blood.

The ionic strength of the formulation may be increased by the addition of sodium chloride. The formulation may comprise from 0.05 to 2 wt% sodium chloride (wherein the weight is based upon the weight of the formulation), or from 0.1 to 1 wt % sodium chloride, or from 0.5 to 0.9 wt% sodium chloride, or about 0.8 wt% sodium chloride.

The inventors have found that by controlling the pH and the osmolality it is possible to prepare stable formulations wherein the concentration of Orellanine is appropriate for intravenous administration.

In an example which is not part of the invention, the pharmaceutical aqueous solution formulation comprises from 0.1 to 40 mg/ml of Orellanine; a phosphate buffer wherein the concentration of the buffer is from 10 to 200 mM; and from 0.1 to 1 wt % sodium chloride; wherein the pH of the formulation is in the range of 7 to 8 and optionally wherein the osmolality of the formulation is greater than 200 mOsm.

In another example which is not part of the invention, the pharmaceutical aqueous solution formulation comprises from 1 to 20 mg/ml of Orellanine; a phosphate buffer wherein the concentration of the buffer is from 10 to 200 mM; and from 0.1 to 1 wt % sodium chloride; wherein the pH of the formulation is in the range of 7 to 8 and optionally wherein the osmolality of the formulation is greater than 200 mOsm.

In another example which is not part of the invention, the pharmaceutical aqueous solution formulation comprises from 5 to 15 mg/ml of Orellanine; a phosphate buffer wherein the concentration of the buffer is from 10 to 200 mM; and from 0.1 to 1 wt % sodium chloride; wherein the pH of the formulation is in the range of 7 to 8 and optionally wherein the osmolality of the formulation is greater than 200 mOsm.

In another example which is not part of the invention, the pharmaceutical aqueous solution formulation comprises from 0.1 to 40 mg/ml of Orellanine; a phosphate buffer chosen from NaH₂PO₄ - Na₂HPO₄ or Citrate - Na₂HPO₄ wherein the concentration of the buffer is from 10 to 200 mM; and from 0.1 to 1 wt % sodium chloride; wherein the pH of the formulation is in the range of 7 to 8 and optionally wherein the osmolality of the formulation is greater than 200 mOsm.

In another example which is not part of the invention, the pharmaceutical aqueous solution formulation comprises from 1 to 20 mg/ml of Orellanine; a phosphate buffer chosen from NaH₂PO₄ - Na₂HPO₄ or Citrate - Na₂HPO₄ wherein the concentration of the buffer is from 10 to 200 mM; and from 0.1 to 1 wt % sodium chloride; wherein the pH of the formulation is in the range of 7 to 8 and optionally wherein the osmolality of the formulation is greater than 200 mOsm.

In another example which is not part of the invention, the pharmaceutical aqueous solution formulation comprises from 5 to 15 mg/ml of Orellanine; a phosphate buffer chosen from NaH₂PO₄ - Na₂HPO₄ or Citrate - Na₂HPO₄ wherein the concentration of the buffer is from 10 to 200 mM; and from 0.1 to 1 wt % sodium chloride; wherein the pH of the formulation is in the range of 7 to 8 and optionally wherein the osmolality of the formulation is greater than 200 mOsm.

In an embodiment, the pharmaceutical aqueous solution formulation comprises from 0.1 to 40 mg/ml of Orellanine; a phosphate buffer wherein the concentration of the buffer is from 40 to 60 mM; and from 0.5 to 0.9 wt % sodium chloride; wherein the pH of the formulation is in the range of 7.2 to 7.5 and optionally wherein the osmolality of the formulation is in the range of from 250 to 350 mOsm.

In another embodiment, the pharmaceutical aqueous solution formulation comprises from 1 to 20 mg/ml of Orellanine; a phosphate buffer wherein the concentration of the buffer is from 40 to 60 mM; and from 0.5 to 0.9 wt % sodium chloride; wherein the pH of the formulation is in the range of 7.2 to 7.5 and optionally wherein the osmolality of the formulation is in the range of from 250 to 350 mOsm.

In another embodiment, the pharmaceutical aqueous solution formulation comprises from 5 to 15 mg/ml of Orellanine; a phosphate buffer wherein the concentration of the buffer is from 40 to 60 mM; and from 0.5 to 0.9 wt % sodium chloride; wherein the pH of the formulation is in the range of 7.2 to 7.5 and optionally wherein the osmolality of the formulation is in the range of from 250 to 350 mOsm.

In another embodiment, the pharmaceutical aqueous solution formulation comprises from 0.1 to 40 mg/ml of Orellanine; a phosphate buffer chosen from NaH₂PO₄ - Na₂HPO₄ or Citrate - Na₂HPO₄ wherein the concentration of the buffer is from 40 to 60 mM; and from 0.5 to 0.9 wt % sodium chloride; wherein the pH of the formulation is in the range of 7.2 to 7.5 and optionally wherein the osmolality of the formulation is in the range of from 250 to 350 mOsm.

In another embodiment, the pharmaceutical aqueous solution formulation comprises from 1 to 20 mg/ml of Orellanine; a phosphate buffer chosen from NaH₂PO₄ - Na₂HPO₄ or Citrate - Na₂HPO₄ wherein the concentration of the buffer is from 40 to 60 mM; and from 0.5 to 0.9 wt % sodium chloride; wherein the pH of the formulation is in the range of 7.2 to 7.5 and optionally wherein the osmolality of the formulation is in the range of from 250 to 350 mOsm.

In another embodiment, the pharmaceutical aqueous solution formulation comprises from 5 to 15 mg/ml of Orellanine; a phosphate buffer chosen from NaH₂PO₄ - Na₂HPO₄ or Citrate - Na₂HPO₄ wherein the concentration of the buffer is from 40 to 60 mM; and from 0.5 to 0.9 wt % sodium chloride; wherein the pH of the formulation is in the range of 7.2 to 7.5 and optionally wherein the osmolality of the formulation is in the range of from 250 to 350 mOsm.

The formulation may be prepared by a method comprising steps of:
a) adding a buffer to an aqueous solution of Orellanine;
b) stirring; and
c) adjusting the pH by addition of additional buffer, by addition of base (e.g. sodium hydroxide), and/or by addition of acid (e.g. hydrochloride acid).

Alternatively, the formulation may be prepared by a method comprising steps of:
a) adding Orellanine (optionally as an aqueous solution) to a buffer;
b) stirring; and
c) adjusting the pH by addition of additional buffer, by addition of base (e.g. sodium hydroxide), and/or by addition of acid (e.g. hydrochloride acid).

The steps of stirring and adjusting the pH may be repeated several times to ensure that the final pH is within the chosen range. The inventors have observed that the pH of the formulation may change upon stirring such that pH adjustment is needed to provide a pH within the chosen range and to provide a stable formulation.

The buffer may be prepared by combining the buffer components (e.g. as NaH₂PO₄ and Na₂HPO₄, or sodium citrate and Na₂HPO₄) with water for injection and with sodium chloride.

The formulation may be filtered (e.g. through a 0.22 µm PVDF filter) before it is packaged. The formulation may be packaged in conventional vessels, such as glass injection vials. The vials may be stoppered and sealed with flip off caps.

The formulation may be used in a method of treating cancer such as renal cancer. The formulation may be used for the treatment of patients suffering from or susceptible to renal cell carcinoma. The treatment may be used to treat a tumor that is localized to one or both of the patient's kidneys. Alternatively, the renal cell carcinoma may have metastasized such that at least one renal cell carcinoma tumor is present in at least one non-kidney tissue.

The formulation is suitable for administration by intravenous injection.

The formulation may be administered in a single dose, in a series of daily doses, or in an intermittent dosing format (e.g. a plurality of doses or dose sequences administered between 1 and about 30 days apart, between 1 and about 14 days apart, or between 1 and about 7 days apart).

When administered in a single dose, a dose of between about 1 mg/kg and about 100 mg/kg of Orellanine may be administered to the patient, or a dose of between about 2 mg/kg and about 25 mg/kg of Orellanine, or a dose of between about 5 mg/kg and about 15 mg/kg of Orellanine.

The formulation may be administered in two or more doses. The doses may be administered daily or intermittently (e.g. with at least one non-administration day separating sequential doses). When administered in a plurality of doses, a dose of between about 0.5 mg/kg and about 10 mg/kg of Orellanine may be administered to the patient, or a dose of between about 1 mg/kg and 5 mg/kg, or a dose of about 2 mg/kg.

If sequential doses are administered intermittently, the sequential doses may be administered between two and seven days apart. The formulation may be administered to the patient in three, four, five or six or more doses and wherein each dose is administered between three and five days apart. The formulation may be administered to the patient in four, five, or six or more doses administered between three and four days apart, wherein each dose comprises between about 1 mg/kg to about 20 mg/kg of Orellanine, or about 2 to about 10 mg/kg of Orellanine, or about 5 mg/kg of Orellanine.

A daily dose of the formulation may be administered for at least two days. Typical daily doses administered to patients are between 0.1 and 10 mg/kg, or between 1 and 5 mg/kg, or about 2 mg/kg. Therapeutic protocols may comprise daily administration of the formulation between 5 and about 30 days, or preferably between 10 and 20 days, or most preferably about 14 days.

In certain instances, it may be desirable to conduct a plurality of intermittent administration protocols, daily administration protocols, or a combination thereof, as described above, in combination with rest and/or recovery periods. Thus, in certain instances, it may be desirable to administer the formulation according to a daily or intermittent administration method, measure the tumor response to the therapy, and then conduct subsequent daily or intermittent administration therapies as necessary to eliminate or further reduce the size of the renal cancer tumors. Such administration strategies are well known to the person with normal skills in the field of oncology.

In one embodiment of the present invention a patient suffering from renal cell carcinoma may be treated with the formulation by daily injections of about 0.5 - 5mg Orellanine/kg b.w., most preferably about 2 mg Orellanine/kg b.w., for about 7 - 21 consecutive days, or about 14 consecutive days. One to 5 hours after each daily injection of the formulation, most preferably about 2 hours after such injection, the patient is subjected to hemodialysis for 1-5 h, most preferably about 2 h, in order to eliminate any Orellanine that has not been taken up into tumor tissue and thereby minimize any undesired side effects that might occur in the extracellular space.

The preferred doses and dose regimes described above are based on a human being weighing 70 kg and suffering from renal cell carcinoma with a tumor burden of about 1 kg. However, as is readily known to the worker with normal skills in the field of cancer medicine, such preferred doses and dose regimes are governed to a large extent by patient characteristics such as age, sex, weight, general condition and, above all, the individual patient's tumor burden and response to the treatment. As always, the ultimate responsibility for choosing the proper dose and treatment strategy lies with the physician in charge of the patient.

The invention will now be described by reference to examples which are not intended to be limiting of the invention:

### EXAMPLES

### Solubility study with buffer (pH dependent solubility testing)

A phase solubility study was performed for different buffers to analyse the effect of pH on the solubility of Orellanine. Four buffers were tested:
- Acetate buffer pH 4.5 at 50mM - prepared from 1.81g sodium acetate and 14ml of acetic acid (2N), adjusted up to 1l with water for injection (WFI).
- Phosphate buffer pH 5.8 at 50mM - prepared from 92ml of NaH₂PO₄ and 8ml of Na₂HPO₄, adjusted to 200ml with WFI.
- Phosphate buffer pH 6.8 at 50mM - prepared from 51ml of NaH₂PO₄ and 49ml of Na₂HPO₄, adjusted to 200ml with WFI.
- Phosphate buffer pH 7.4 at 50mM - prepared from 19ml of NaH₂PO₄ and 81ml of Na₂HPO₄, adjusted to 200ml with WFI.

The pH of each buffer was adjusted with a pH meter and sodium hydroxide or hydrochloric acid at 0.2M.

The solubility of Orellanine in each buffer was tested by weighing 40mg of Orellanine into a vial, adding 2ml of buffer, stirring the suspension for 1 hour, and then stirring at 400rpm for 24 hours at room temperature. Each suspension was centrifuged at 5300rpm for 15 minutes. The supernatant was filtered with a 0.45µm nylon filter. Table 1 shows the outcome of the testing:

**Table 1**

| | Appearance of supernatant | pH | Osmolality of supernatant (mOsm) | Concentration of Orellanine in supernatant (mg/ml) |
|---|---|---|---|---|
| Acetate buffer pH 4.5 at 50mM | Colourless solution | 4.66 | 119 | 0.0024 |
| Phosphate buffer pH 5.8 at 50mM | Colourless solution | 5,84 | 102 | 0.0299 |
| Phosphate buffer pH 6.8 at 50mM | Yellowish solution | 6.79 | 119 | 0.6218 |
| Phosphate buffer pH 7.4 at 50mM | Yellowish solution | 7.08 | 135 | 1.7150 |

The maximum concentration of Orellanine achieved was 1.7mg/ml with phosphate buffer pH 7.4 at 50mM.

### Solubility study with different buffer concentrations at pH 7.4

The impact of buffer concentration was assessed. Three buffers were used:
- Phosphate buffer pH 7.4 at 25mM - prepared from 0.95ml of NaH₂PO₄ (0.1M) and 4.05ml of Na₂HPO₄ (0.1M), adjusted to 20ml with WFI.
- Phosphate buffer pH 7.4 at 50mM - prepared from 19ml of NaH₂PO₄ (0.1M) and 81ml of Na₂HPO₄ (0.1M), adjusted to 200ml with WFI.
- Phosphate buffer pH 7.4 at 100mM - prepared from 38ml NaH₂PO₄ (0.1M) and 162ml of Na₂HPO₄ (0.1M), adjusted to 200ml with WFI.

The pH of each buffer was adjusted with a pH meter and sodium hydroxide or hydrochloric acid at 0.2M.

The solubility of Orellanine in each buffer was tested by weighing 20mg of Orellanine into a 5ml vial, adding 2ml of buffer, stirring the suspension for 3 hours, and then adjusting the pH to 7.4 with sodium hydroxide or hydrochloric acid at 0.2M. The suspension was then stirred at 400rpm for 24 hours at room temperature. Each suspension was filtered through a 0.2µm PVDF syringe filter. Table 2 shows the outcome of the testing:

**Table 2**

| | Appearance after 24 hours of stirring | Appearance after filtration | pH | Osmolality (mOsm) | Concentration of Orellanine (mg/ml) | Purity of Orellanine |
|---|---|---|---|---|---|---|
| NaH₂PO₄ - Na₂HPO₄ pH 7.4 25mM | Yellow solution with particles in suspension | Yellow solution | 7.29 | 92 | 3.68 | 99.3 |
| NaH₂PO₄ - Na₂HPO₄ pH 7.4 50mM | Yellow solution | Yellow solution | 7.33 | 150 | 4.88 | 100 |
| NaH₂PO₄ - Na₂HPO₄ pH 7.4 100mM | Yellow solution | Yellow solution | 7.30 | 242 | 4.91 | 99.4 |

The solubility ranged from 3.7 to 4.9mg/ml for these formulations. 50mM seems to be the optimum concentration of buffer from a solubility and purity perspective.

### Solubility study with different buffer salts at pH 7.4

Four buffers at pH 7.4 and 50mM were assessed:
- Phosphate buffer pH 7.4 at 50mM - prepared from 19ml of NaH₂PO₄ (0.1M) and 81ml of Na₂HPO₄ (0.1M), adjusted to 200ml with WFI.
- Phosphate buffer pH 7.4 at 50mM - prepared from 28.1ml of KH₂PO₄ (0.2M) and 21.9ml of NaOH (0.2M) adjusted to 200ml with WFI.
- Tris-HCl buffer pH 7.4 at 50mM - prepared from 54.7ml of tris(hydroxymethyl)aminomethane (0.1M) and 45.3ml of hydrochloric acid (0.1M), adjusted to 200ml with WFI.
- Citrate phosphate buffer pH 7.4 at 50mM - prepared from 2.4ml of citric acid monohydrate (0.2M) and 47.6ml of Na₂HPO₄ (0.2M), adjusted to 200ml with WFI.

The pH of each buffer was adjusted with a pH meter and sodium hydroxide or hydrochloric acid at 0.2M.

The solubility of Orellanine in each buffer was tested by weighing 20mg of Orellanine into a 5ml vial, adding 2ml of buffer, stirring the suspension for 3 hours, and then adjusting the pH to 7.4 with sodium hydroxide or hydrochloric acid at 0.2M. The suspension was then stirred at 400rpm for 24 hours at room temperature. Each suspension was filtered through a 0.2µm PVDF syringe filter. Table 3 shows the outcome of the testing:

**Table 3**

| | Appearance after 24 hours of stirring | Appearance after filtration | pH | Osmolality (mOsm) | Concentration of Orellanine (mg/ml) | Purity of Orellanine |
|---|---|---|---|---|---|---|
| NaH₂PO₄ - Na₂HPO₄ pH 7.4 | Yellow solution | Yellow solution | 7.33 | 150 | 4.88 | 100 |
| KH₂PO₄ - NaOH pH 7.4 | Yellow solution | Yellow solution | 7.40 | 108 | 4.56 | 99.4 |
| Tris-HCl pH 7.4 | Yellow solution | Yellow solution | 7.49 | 95 | 4.59 | 99.5 |
| Citrate - Na₂HPO₄ pH 7.4 | Yellow solution | Yellow solution | 7.32 | 157 | 5.44 | 99.5 |

The best solubility was provided by the NaH₂PO₄ - Na₂HPO₄ and Citrate - Na₂HPO₄ buffers.

### Solubility study with different ionic strengths at pH 7.4

The ionic strength of the NaH₂PO₄ - Na₂HPO₄ buffer was adjusted by adding sodium chloride. The ionic strength of the NaH₂PO₄ - Na₂HPO₄ buffer at 50mM was estimated at around 130mM. The proportion of sodium chloride in added to the buffers was 0.41%, 0.98%, and 2.15%.

The solubility of Orellanine in each buffer was tested by weighing 20mg of Orellanine into a 5ml vial, adding 2ml of buffer, stirring the suspension for 3 hours, and then adjusting the pH to 7.4 with sodium hydroxide or hydrochloric acid at 0.2M. The suspension was then stirred at 400rpm for 24 hours at room temperature. Each suspension was filtered through a 0.2µm PVDF syringe filter. Table 4 shows the outcome of the testing:

**Table 4**

| | Appearance after 24 hours of stirring | Appearance after filtration | pH | Osmolality (mOsm) | Concentration of Orellanine (mg/ml) | Purity of Orellanine |
|---|---|---|---|---|---|---|
| NaH₂PO₄ - Na₂HPO₄ pH 7.4 at 50mM plus 0.41% NaCl | Yellow solution | Yellow solution | 7.26 | 205 | 5.31 | 99.4 |
| NaH₂PO₄ - Na₂HPO₄ pH 7.4 at 50mM plus 0.98% NaCl | Yellow solution | Yellow solution | 7.19 | 328 | 5.90 | 99.4 |
| NaH₂PO₄ - Na₂HPO₄ pH 7.4 at 50mM plus 2.15% NaCl | Yellow solution | Yellow solution | 7.30 | 595 | 6.43 | 99.4 |

Increasing the ionic strength increased the osmolality of the solution and promoted Orellanine solubility.

### Formulation adjustment

Two buffers were selected: NaH₂PO₄ - Na₂HPO₄ pH 7.4 and Citrate - Na₂HPO₄ pH 7.4 at 50mM, each with addition of 0.70% sodium chloride. Adding 0.70% sodium chloride provides a solution close to isotonicity.

Formulations were prepared by weighing 40mg of Orellanine into a 5ml vial, adding 2ml of buffer, stirring the suspension for 1 hour, adjusting the pH to 7.4 with sodium hydroxide (0.2M), stirring the suspension at 400rpm for 24 hours at room temperature, and adjusting the pH to 7.4 with sodium hydroxide (0.2M). Each suspension was filtered through a 0.2µm PVDF syringe filter. Table 5 shows the properties of the formulations:

**Table 5**

| | Appearance after 24 hours of stirring | Appearance after filtration | pH | Osmolality (mOsm) | Concentration of Orellanine (mg/ml) | Purity of Orellanine |
|---|---|---|---|---|---|---|
| NaH₂PO₄ - Na₂HPO₄ pH 7.4 at 50mM plus 0.70% NaCl | White to off-white suspension | Yellow solution | 7.29 | 312 | 11.1 | 99.5 |
| Citrate - Na₂HPO₄ pH 7.4 at 50mM plus 0.70% NaCl | White to off-white suspension | Yellow solution | 7.24 | 315 | 10.5 | 99.4 |

Solubility of greater than 10mg/ml was achieved by twice adjusting the pH to 7.4: firstly after 1 hour of stirring and again after an overnight stirring.

## Claims

1. A pharmaceutical aqueous solution formulation comprising
from 0.1 to 40 mg/ml of Orellanine; and
a buffer;
wherein the pH of the formulation is in the range of 7 to 8, and
wherein the concentration of the buffer is from 40 to 80 mM.

2. The formulation according to claim 1, comprising from 1 to 20 mg/ml of Orellanine.

3. The formulation according to claim 2, comprising from 5 to 15 mg/ml of Orellanine.

4. The formulation according to any preceding claim, wherein the pH of the formulation is in the range of 7.1 to 7.6.

5. The formulation according to claim 4, wherein the pH of the formulation is in the range of 7.2 to 7.5.

6. The formulation according to any preceding claim, wherein the concentration of the buffer is from 40 to 60 mM.

7. The formulation according to any preceding claim wherein the buffer is a phosphate buffer, optionally wherein the buffer is NaH₂PO₄ - Na₂HPO₄ or Citrate - Na₂HPO₄.

8. The formulation according to any preceding claim, wherein the osmolality of the formulation is greater than 200 mOsm.

9. The formulation according to any preceding claim, wherein the osmolality of the formulation is in the range of from 250 to 350 mOsm.

10. The formulation according to any preceding claim, comprising sodium chloride, optionally wherein the formulation comprises from 0.1 to 1 wt % sodium chloride.

11. A formulation according to any preceding claim, for use in a method of treating cancer, optionally wherein the cancer is renal cancer and/or wherein the method comprises intravenously administering the formulation to a subject in need thereof.

12. The formulation for use according to claim 11, wherein the method comprises providing the formulation to a subject in need thereof for at least two days, wherein the daily dose is between 0.1 and 10 mg/kg of body weight.

13. A method for preparing a formulation according to any one of claims 1 to 10, comprising steps of:
a) adding a buffer to an aqueous solution of Orellanine, or adding Orellanine, optionally as an aqueous solution, to a buffer;
b) stirring; and
c) adjusting the pH by addition of additional buffer, by addition of base (e.g. sodium hydroxide), and/or by addition of acid (e.g. hydrochloride acid).

14. A method according to claim 13, wherein steps (b) and (c) are carried out at least twice.

15. A kit for intravenous administration, comprising a container packaging the formulation according to any one of claims 1 to 10, and optionally further comprising information for providing the formulation to a subject in need thereof.

## Patentansprüche

1. Pharmazeutische wässrige Lösungsformulierung,
umfassend 0,1 bis 40 mg/ml Orellanin; und
einen Puffer;
wobei der pH der Formulierung in dem Bereich von 7 bis 8 ist und
wobei die Konzentration des Puffers von 40 bis 80 mM ist.

2. Formulierung nach Anspruch 1, umfassend 1 bis 20 mg/ml Orellanin.

3. Formulierung nach Anspruch 2, umfassend 5 bis 15 mg/ml Orellanin.

4. Formulierung nach einem der vorstehenden Ansprüche, wobei der pH der Formulierung in dem Bereich von 7,1 bis 7,6 ist.

5. Formulierung nach Anspruch 4, wobei der pH der Formulierung in dem Bereich von 7,2 bis 7,5 ist.

6. Formulierung nach einem der vorstehenden Ansprüche, wobei die Konzentration des Puffers von 40 bis 60 mM ist.

7. Formulierung nach einem der vorstehenden Ansprüche, wobei der Puffer ein Phosphatpuffer ist, optional wobei der Puffer NaH₂PO₄ - Na₂HPO₄ oder Citrate - Na₂HPO₄ ist.

8. Formulierung nach einem der vorstehenden Ansprüche, wobei die Osmolalität der Formulierung größer als 200 mOsm ist.

9. Formulierung nach einem der vorstehenden Ansprüche, wobei die Osmolalität der Formulierung in dem Bereich von 250 bis 350 mOsm ist.

10. Formulierung nach einem der vorstehenden Ansprüche, umfassend Natriumchlorid, optional wobei die Formulierung von 0,1 bis 1 Gew.-% Natriumchlorid umfasst.

11. Formulierung nach einem der vorstehenden Ansprüche zur Verwendung bei einem Verfahren zur Behandlung von Krebs, optional wobei der Krebs Nierenkrebs ist und/oder wobei das Verfahren ein intravenöses Verabreichen der Formulierung an ein Subjekt umfasst, das diese benötigt.

12. Formulierung zur Verwendung nach Anspruch 11, wobei das Verfahren ein Bereitstellen der Formulierung für ein Subjekt, das diese benötigt, über mindestens zwei Tage umfasst, wobei die Tagesdosis zwischen 0,1 und 10 mg/kg Körpergewicht ist.

13. Verfahren zur Herstellung einer Formulierung nach einem der Ansprüche 1 bis 10, umfassend folgende Schritte:
a) Hinzufügen eines Puffers zu einer wässrigen Orellanin-Lösung oder Hinzufügen von Orellanin, optional als wässrige Lösung, zu einem Puffer;
b) Rühren; und
c) Einstellen des pHs durch Hinzufügen von zusätzlichem Puffer, durch Hinzufügen von Base (z. B. Natriumhydroxid) und/oder durch Hinzufügen von Säure (z. B. Salzsäure).

14. Verfahren nach Anspruch 13, wobei Schritt (b) und (c) mindestens zweimal durchgeführt werden.

15. Kit zur intravenösen Verabreichung, umfassend einen Behälter, der die Formulierung nach einem der Ansprüche 1 bis 10 umschließt, und optional ferner umfassend Informationen zum Bereitstellen der Formulierung an ein Subjekt, das diese benötigt.

## Revendications

1. Formulation pharmaceutique en solution aqueuse comprenant
0,1 à 40 mg/ml d'orellanine ; et
un tampon ;
dans laquelle le pH de la formulation est compris dans la plage de 7 à 8, et
dans laquelle la concentration du tampon est de 40 à 80 mM.

2. Formulation selon la revendication 1, comprenant de 1 à 20 mg/ml d'orellanine.

3. Formulation selon la revendication 2, comprenant de 5 à 15 mg/ml d'orellanine.

4. Formulation selon une quelconque revendication précédente, dans laquelle le pH de la formulation est compris dans la plage de 7,1 à 7,6.

5. Formulation selon la revendication 4, dans laquelle le pH de la formulation est compris dans la plage de 7,2 à 7,5.

6. Formulation selon une quelconque revendication précédente, dans laquelle la concentration du tampon est de 40 à 60 mM.

7. Formulation selon une quelconque revendication précédente, dans laquelle le tampon est un tampon phosphate, éventuellement dans laquelle le tampon est NaH₂PO₄ - Na₂HPO₄ ou Citrate - Na₂HPO₄.

8. Formulation selon une quelconque revendication précédente, dans laquelle l'osmolalité de la formulation est supérieure à 200 mOsm.

9. Formulation selon une quelconque revendication précédente, dans laquelle l'osmolalité de la formulation est comprise dans la plage de 250 à 350 mOsm.

10. Formulation selon une quelconque revendication précédente, comprenant du chlorure de sodium, éventuellement dans laquelle la formulation comprend de 0,1 à 1 % en poids de chlorure de sodium.

11. Formulation selon une quelconque revendication précédente, destinée à être utilisée dans un procédé de traitement d'un cancer, éventuellement dans laquelle le cancer est un cancer rénal et/ou dans laquelle le procédé comprend l'administration intraveineuse de la formulation à un sujet qui en a besoin.

12. Formulation destinée à être utilisée selon la revendication 11, dans laquelle le procédé comprend l'administration de la formulation à un sujet qui en a besoin pendant au moins deux jours, dans laquelle la dose quotidienne est comprise entre 0,1 et 10 mg/kg de poids corporel.

13. Procédé pour la préparation d'une formulation selon l'une quelconque des revendications 1 à 10, comprenant les étapes de :
a) ajout d'un tampon à une solution aqueuse d'orellanine, ou ajout d'orellanine, éventuellement sous forme de solution aqueuse, à un tampon ;
b) agitation ; et
c) ajustement du pH par ajout de tampon supplémentaire, par ajout de base (par exemple, l'hydroxyde de sodium) et/ou par ajout d'acide (par exemple, l'acide chlorhydrique).

14. Procédé selon la revendication 13, dans lequel les étapes (b) et (c) sont réalisées au moins deux fois.

15. Kit pour administration intraveineuse, comprenant un récipient contenant la formulation selon l'une quelconque des revendications 1 à 10, et comprenant en outre, éventuellement, des informations pour administrer la formulation à un sujet qui en a besoin.
